# EUROPEAN PATENT APPLICATION

(11) **EP 3 446 675 A1**
(43) Date of publication of application: **27.02.2019**
(21) Application number: 18160061.0
(22) Date of filing: 05.03.2018
(51) Int. Cl.: A61K 8/25, A61K 8/26, A61Q 1/12, A61K 8/58, A61K 8/64, A61K 8/02, A61K 8/19

(54) **HYDROPHOBIC AND OLEOPHOBIC COSMETIC PIGMENT POWDER WITH EXCELLENT SKIN-FITTING PROPERTY AS WELL AS PREPARATION METHOD AND APPLICATION THEREOF**

(30) Priority: 22.08.2017 CN 201710723359
(71) Applicant: Shanghai Sciencoo Biotech Co., Ltd., 200435 Shanghai (CN)
(72) Inventor: KANG, Rongmin, Shanghai, 200435 (CN); XIE, Xu, Shanghai, 200435 (CN); QIN, Jihua, Shanghai, 200435 (CN)
(74) Representative: Dargiewicz, Joanna

(57) **Abstract**

The present invention discloses a hydrophobic and oleophobic cosmetic pigment powder, including pigment powder and a skin-fitting treatment agent. The skin-fitting treatment agent can perform surface compounding treatment on the pigment powder and includes fluorine-containing silane and hydrogenated lecithin. The hydrophobic and oleophobic cosmetic pigment powder has excellent hydrophobic and oleophobic properties, has excellent pickable property and skin-fitting property, and makes skin elastic, soft and smooth. The perfluorinated silane treatment agent in the present invention has a carbon number less than or equal to 8, is safe, skin-friendly and nontoxic, and can achieve effects of nourishing skin and easily removing makeup due to addition of pure plant ingredients with excellent skin affinity.

## Description

### FIELD OF THE INVENTION

The present invention relates to the field of cosmetics, and particularly relates to a hydrophobic and oleophobic cosmetic pigment powder and a preparation method thereof.

### BACKGROUND OF THE INVENTION

With the increasing improvement of a living standard, demands of people for cosmetics are increased rapidly. In the past, most of foundation cream/liquid foundation have a waterproof function, but rarely have a grease-proof function. During use, grease, sweat and the like secreted by skin are easy to destroy makeup look. Drop and disturbance of the makeup look may be caused to different degrees according to differences of skins of different people.

In recent years, fluorine-containing silane is used seldom in the industry of cosmetics. For example, JP2008037813 and JP200813792 disclose an application of the fluorine-containing silane in surface treatment of the cosmetic pigment powder for achieving hydrophobic and oleophobic effects. The powder is poor in skin feeling and has poor skin fitting after use, and meanwhile, the cosmetics have a color floating problem. CN201610003469 solves the above color floating problem, and discloses a hydrophobic and oleophobic cosmetic powder including nano-silicon sol and titanium dioxide treated by perfluorooctyl triethoxysilane. Although the cosmetic raw powder treated by using the fluorine-containing silane currently has excellent hydrophobic and oleophobic properties and may maintain relatively long-lasting makeup, the powder is poor in pickable property and skin fitting property, and the makeup is difficult to be removed. CN201080039592.8 discloses a cosmetic composition with an ultraviolet shielding effect and a sebum curing capability. The composition includes one or more than two of particulate titanium oxide, magnesium oxide, calcium oxide, magnesium hydroxide and calcium hydroxide and is subjected to oleophilic treatment by an oleophilic treatment agent. The oleophilic treatment agent is formed by alkyl alkoxy silane represented by a general formula (1), reactive organo-siloxane represented by a general formula (2), acylated amino acid, polyolefin, hydrogenated lecithin, dextrin aliphatic ester, compounds containing perfluoroalkyl groups or perfluoroalkylpolyether groups and one compound or a mixture of more than two compounds of saturated or unsaturated fatty acids with the carbon number of 12 to 22 including forms of salts thereof. The compound 1 is represented as (CₙH₂ₙ₊₁)ₐSi(OCₘH2m+1)_{b}; in the formula 1, n represents an integer of 1-18, m represents an integer of 1-3, a and b respectively represent an integer of 1-3, and a+b is equal to 4. The compound 2 is represented as (R¹₃SiO)Si(R¹₂SiO)ₚ(SiR²₃); in the formula 2, all of R¹ independently represent lower alkyl or hydrogen atoms with the carbon number of 1 to 4 respectively, R² represents any one of amino, hydrogen atom, hydroxyl and lower alkoxy with the carbon number of 1 to 4, and P is an integer of 1-300. The carbon number of fluorinated alkyl compounds used in the patent is 12 to 22, and fluorinated alkyl compounds with the carbon number greater than 8 have biotoxicity. Hydrophobic and oleophilic powder is obtained in CN201080039592.8, is poor in makeup retaining property, and mainly aims to shield ultraviolet ray.

### SUMMARY OF THE INVENTION

In order to solve the above technical problem, a purpose of the present invention is to provide a hydrophobic and oleophobic cosmetic pigment powder. The powder not only has excellent hydrophobic and oleophobic properties, but also has excellent pickable property and skin fitting property, and makes skin elastic, soft and smooth. A perfluorinated silane treatment agent in the present invention has a carbon number less than or equal to 8, and is safe, skin-friendly and nontoxic. Effects of nourishing skin and removing the makeup can be achieved since pure plant ingredients with excellent skin affinity are added.

Another purpose of the present invention is to provide a preparation method of the hydrophobic and oleophobic cosmetic pigment powder.

The present invention is realized through the following technical solution:

The hydrophobic and oleophobic cosmetic pigment powder includes a pigment powder and a skin-fitting treatment agent. The skin-fitting treatment agent can perform surface compounding treatment on the pigment powder and includes fluorine-containing silane and hydrogenated lecithin.

Further preferably, the fluorine-containing silane and the hydrogenated lecithin in the skin-fitting treatment agent in the present invention are used together according to a weight ratio of 1:1-15:1, further preferably (1-5):1, and preferably 3:1.

The hydrophobic and oleophobic cosmetic pigment powder includes 0.1-10.0% of the fluorine-containing silane, 0.1-10.0% of the hydrogenated lecithin, and the balance of pigment powder.

The pigment powder in the hydrophobic and oleophobic cosmetic pigment powder includes but not limited to one or a mixture of more of titanium dioxide, iron oxide yellow, iron oxide red, iron oxide black, talc, mica, ultramarines and chromium oxide green.

A structure of the fluorine-containing silane in the skin-fitting treatment agent is R_{f}-(CH₂)₂-Si-(OCₘH₂ₘ₊₁)₃, wherein R_{f} is CₙF₂ₙ₊₁, n is more than or equal to 1 and less than or equal to 6, n is an integer, and m is 1 or 2.

Further preferably, the fluorine-containing silane is perfluorooctyl triethoxysilane.

The hydrogenated lecithin in the skin-fitting treatment agent has a biological name of phosphatidylcholine, is honored as "the third nutrient" tied with protein and vitamin, and has a generic name of Lecithin. All phosphorus-containing lipids discovered in the lecithin are classified as "phospholipid", and the phospholipid, glycolipids and (aphingolipid) sphingolipids represent polar lipids in the lecithin, which are essential ingredients of all cell membranes. The lecithin applied to the field of cosmetics is extracted from soybeans, is biodegradable and is an ecologically environmental-friendly product. The lecithin is honored as an "edible cosmetic". The hydrogenated lecithin is used as a surface treatment agent of the cosmetic powder, and has similar biochemical property to skin because of the existence of the lecithin. Therefore, the treated cosmetic powder has excellent compatibility to the skin.

In a technical solution, the hydrophobic and oleophobic cosmetic pigment powder in the present invention is composed of the following substances in percentage by weight:
99.64% of talcum powder treated by 6% of perfluorooctyl triethoxysilane+2% of hydrogenated lecithin;
0.24% of iron oxide yellow treated by 6% of perfluorooctyl triethoxysilane+2% of hydrogenated lecithin;
0.10% of iron oxide red treated by 6% of perfluorooctyl triethoxysilane+2% of hydrogenated lecithin; and
0.02% of iron oxide black treated by 6% of perfluorooctyl triethoxysilane+2% of hydrogenated lecithin.
In a technical solution, the hydrophobic and oleophobic cosmetic pigment powder in the present invention is composed of the following substances in percentage by weight:
53.25% of talcum powder treated by 6% of perfluorooctyl triethoxysilane+2% of hydrogenated lecithin;
41.32% of mica powder treated by 6% of perfluorooctyl triethoxysilane+2% of hydrogenated lecithin;
2.36% of titanium dioxide treated by 6% of perfluorooctyl triethoxysilane+2% of hydrogenated lecithin;
0.71% of iron oxide yellow treated by 6% of perfluorooctyl triethoxysilane+2% of hydrogenated lecithin; and
2.36% of iron oxide red treated by 6% of perfluorooctyl triethoxysilane+2% of hydrogenated lecithin.

In a technical solution, the hydrophobic and oleophobic cosmetic pigment powder in the present invention is composed of the following substances in percentage by weight:
91.8% of titanium dioxide treated by 6% of perfluorooctyl triethoxysilane+2% of hydrogenated lecithin;
5.4% of iron oxide yellow treated by 6% of perfluorooctyl triethoxysilane+2% of hydrogenated lecithin;
1.8% of iron oxide red treated by 6% of perfluorooctyl triethoxysilane+2% of hydrogenated lecithin; and
1.0% of iron oxide black treated by 6% of perfluorooctyl triethoxysilane+2% of hydrogenated lecithin.

The surface compounding treatment for the pigment powder by the skin-fitting treatment agent may be performed by a conventional dry method or a wet process.

An application of the hydrophobic and oleophobic cosmetic pigment powder of the present invention in the cosmetics is disclosed.

According to the application in the present invention, the cosmetics are make-up products with functions of covering skin flaws and modifying a skin color, such as loose powder, pressed powder, blusher, liquid foundation, foundation cream and the like. The hydrophobic and oleophobic cosmetic pigment powder with excellent skin-fitting property prepared in the present invention achieves a physical covering effect in the make-up products and can immediately modify the skin flaws and create a perfect makeup effect. Moreover, because of the existence of the hydrogenated lecithin with excellent skin-fitting property derived from pure plants, the hydrophobic and oleophobic cosmetic pigment powder can nourish skin and build a concept of skin care makeup of the plants.

The cosmetics may also be skin care products with a whitening function, such as whitening cream, whitening lotion and the like. The hydrophobic and oleophobic cosmetic pigment powder with excellent skin-fitting property prepared in the present invention achieves effects of physically whitening and nourishing the skin for a long time in the skin care products, can immediately whiten the skin and nourish the skin, and is beneficial to matching with long-lasting whitening ingredients to build a concept of cosmetics with dual effects of whitening and nourishing.

The present invention further provides a preparation method of the hydrophobic and oleophobic cosmetic pigment powder. The preparation method includes the following steps:
step (1), preparing a skin-fitting treatment solution: dissolving the fluorine-containing silane and the hydrogenated lecithin in ethanol or polyhydric alcohols to prepare the treatment solution;
step (2), spraying the treatment solution onto a surface of the pigment powder while stirring at high speed, uniformly mixing and discharging, heating and drying; and
step (3), performing air jet milling to obtain the powder.

Further preferably, the preparation method of the hydrophobic and oleophobic cosmetic pigment powder includes the following steps:
step (1), preparing a skin-fitting treatment agent: dissolving the fluorine-containing silane and the hydrogenated lecithin in ethanol or polyhydric alcohols with an amount of 1-1.5 times of a total amount of a compound treatment agent while stirring at a high speed of 2000-20000 revolutions per minute to prepare a treatment solution;
step (2), spraying the treatment solution onto a surface of the pigment powder while stirring at the high speed of 2000-20000 revolutions per minute, uniformly mixing and discharging, heating at 100-150° and drying for 1-5 hours; and
step (3), performing air jet milling: treating the powder by adopting high-pressure air of 7-8 Mpa, thereby obtaining the powder with a mean particle size of 0.1-5µm.

The polyhydric alcohols in the step (1) include but not limited to glycerin, isopropanol, ethylene glycol, propanetriol and the like.

### BENEFICIAL EFFECTS

1. The hydrophobic and oleophobic cosmetic pigment powder in the present invention is compounded by the fluorine-containing silane and the hydrogenated lecithin and overcomes the defects of no skin-fitting property and difficult makeup removal in the powder, prepared by fluoridation, having excellent hydrophobic and oleophobic properties. Since the hydrogenated lecithin is derived from pure plants and has excellent skin-fitting property, the powder can enhance the skin-fitting property and nourishing property to skin on premise of ensuring good hydrophobic and oleophobic properties, and is also easier to remove the makeup and excellent in skin feeling, thereby making skin silk-like elastic, soft and smooth;
2. a ratio of the hydrogenated lecithin to the fluorine-containing silane in the present invention is obtained by virtue of massive screening, and only in the ratio in the present invention, the prepared hydrophobic and oleophobic cosmetic pigment powder may achieve the effects of skin fitting and easiness of makeup removal;
3. a process for treating the pigment powder by using the skin-fitting treatment agent in the present invention is simple, easy to operate and easy to realize industrialized large-scaleproduction;
4. the treatment agent compounded by the hydrogenated lecithin and the fluorine-containing silane in the present invention can reduce surface property differences among different types of pigment powder, so that the amount of the fluorine-containing silane coated on surfaces of the different types of pigment powder tends to be consistent; and therefore, when multiple types of pigment powder are applied to the same cosmetic formula, the powder can be uniformly scattered to avoid color floating, and the cosmetic formula can also be more skin-fitting and easy to remove the makeup;
5. silicon dioxide used in CN201610003469 improves the color floating by enabling the surface properties to tend to be unified; and after compounding treatment of the hydrogenated lecithin is added in the present invention, the compatibility of different types of powder is improved, and the obtained composite colored powder has excellent compatibility to the formula; and therefore, the color floating may be improved; and
6. the carbon number of the fluorinated alkyl compound used in CN201080039592.8 is 12 to 22, and the obtained hydrophobic and oleophilic powder is poor in make-up retaining property and mainly aims to shield ultraviolet ray. The perfluorinated silane compound used in the present invention has the carbon number of 3 to 8 and is a safe and environmental-friendly perfluorinated silane treatment agent; and the obtained hydrophobic and oleophobic powder can achieve the effects of retaining make-up for a long time, realizing affinity and nourishing of the skin, and easily removing the makeup.

### BRIEF DESCRIPTION OF THE DRAWINGS

Fig. 1 is a contact angle test picture of titanium dioxide treated by 6% of perfluorooctyl triethoxysilane+2% of hydrogenated lecithin;
Fig. 2 is a contact angle test picture of iron oxide yellow powder treated by 6% of perfluorooctyl triethoxysilane+2% of hydrogenated lecithin;
Fig. 3 is a contact angle test picture of iron oxide black powder treated by 6% of perfluorooctyl triethoxysilane+2% of hydrogenated lecithin;
Fig. 4 is a contact angle test picture of iron oxide red powder treated by 6% of perfluorooctyl triethoxysilane+2% of hydrogenated lecithin;
Fig. 5 is a contact angle test picture of talcum powder treated by 6% of perfluorooctyl triethoxysilane+2% of hydrogenated lecithin;
Fig. 6 is a contact angle test picture of mica powder treated by 6% of perfluorooctyl triethoxysilane+2% of hydrogenated lecithin;
Fig. 7 is a contact angle test picture of titanium dioxide treated by 6% of perfluorooctyl triethoxysilane+2% of hydrogenated lecithin;
Fig. 8 is a contact angle test picture of iron oxide yellow powder treated by 6% of perfluorooctyl triethoxysilane+2% of hydrogenated lecithin;
Fig. 9 is a contact angle test picture of iron oxide black powder treated by 6% of perfluorooctyl triethoxysilane;
Fig. 10 is a contact angle test picture of iron oxide red powder treated by 6% of perfluorooctyl triethoxysilane;
Fig. 11 is a contact angle test picture of talcum powder treated by 6% of perfluorooctyl triethoxysilane; and
Fig. 12 is a contact angle test picture of mica powder treated by 6% of perfluorooctyl triethoxysilane.

### DETAILED DESCRIPTION OF THE EMBODIMENTS

Specific embodiments of the present invention are described in detail below in combination with drawings. However, it should be understood that, a protection scope of the present invention is not limited by specific embodiments.

Unless otherwise expressly stated, in the whole description and claims, a term "include" or transformations thereof, such as "contain" or "including" and the like, should be understood to include the stated elements or components, rather than excluding other elements or other components.

### Embodiment 1

steps: dissolving 60g of perfluorooctyl triethoxysilane (Dynasylan F8261, Evonik Degussa) and 20g of hydrogenated lecithin (LECIPLART-SOY • 75H, TECHNOBLE) in 100g of ethanol to prepare a treatment solution; spraying the treatment solution into 1kg of titanium dioxide while high-speed stirring, fully mixing the treatment solution and powder and then discharging, and drying at 110° for 3h; and performing air jet milling after discharging, thereby obtaining the titanium dioxide subjected to compounding treatment.

### Embodiment 2

steps: dissolving 60g of perfluorooctyl triethoxysilane (Dynasylan F8261, Evonik Degussa) and 20g of hydrogenated lecithin (LECIPLART-SOY • 75H, TECHNOBLE) in 100g of ethanol to prepare a treatment solution; spraying the treatment solution into 1kg of iron oxide yellow while high-speed stirring, fully mixing the treatment solution and powder and then discharging, and drying at 110° for 3h; and performing air jet milling after discharging, thereby obtaining the iron oxide yellow subjected to compounding treatment.

### Embodiment 3

steps: dissolving 60g of perfluorooctyl triethoxysilane (Dynasylan F8261, Evonik Degussa) and 20g of hydrogenated lecithin (LECIPLART-SOY • 75H, TECHNOBLE) in 100g of ethanol to prepare a treatment solution; spraying the treatment solution into 1kg of iron oxide red while high-speed stirring, fully mixing the treatment solution and powder and then discharging, and drying at 110° for 3h; and performing air jet milling after discharging, thereby obtaining the iron oxide red subjected to compounding treatment.

### Embodiment 4

steps: dissolving 60g of perfluorooctyl triethoxysilane (Dynasylan F8261, Evonik Degussa) and 20g of hydrogenated lecithin (LECIPLART-SOY • 75H, TECHNOBLE) in 100g of ethanol to prepare a treatment solution; spraying the treatment solution into 1kg of iron oxide black while high-speed stirring, fully mixing the treatment solution and powder and then discharging, and drying at 110° for 3h; and performing air jet milling after discharging, thereby obtaining the iron oxide black subjected to compounding treatment.

### Embodiment 5

steps: dissolving 60g of perfluorooctyl triethoxysilane (Dynasylan F8261, Evonik Degussa) and 20g of hydrogenated lecithin (LECIPLART-SOY • 75H, TECHNOBLE) in 100g of ethanol to prepare a treatment solution; spraying the treatment solution into 1 kg of talcum powder while high-speed stirring, fully mixing the treatment solution and powder and then discharging, and drying at 110° for 3h; and performing air jet milling after discharging, thereby obtaining the talcum powder subjected to compounding treatment.

### Embodiment 6

steps: dissolving 60g of perfluorooctyl triethoxysilane (Dynasylan F8261, Evonik Degussa) and 20g of hydrogenated lecithin (LECIPLART-SOY • 75H, TECHNOBLE) in 100g of ethanol to prepare a treatment solution; spraying the treatment solution into 1kg of sericite while high-speed stirring, fully mixing the treatment solution and powder and then discharging, and drying at 110° for 3h; and performing air jet milling after discharging, thereby obtaining the sericite subjected to compounding treatment.

### Embodiment 7 (loose powder and pressed powder)

The powder prepared in embodiments 2, 3, 4 and 5 is added into a formula shown in Table 1 to prepare the loose powder or pressed powder.

**Table 1 Formula composition of loose powder**

| Phase | Component name | Mass percentage % | Producer/supplier |
|---|---|---|---|
| A | Boron nitride | 3.00 | Combined micro powder |
| | Talcum powder treated by 6% of perfluorooctyl triethoxysilane+2% of hydrogenated lecithin (embodiment 5) | 82.30 | Self-produced |
| | Iron oxide yellow treated by 6% of perfluorooctyl triethoxysilane+2% of hydrogenated lecithin (embodiment 2) | 0.20 | Self-produced |
| | Iron oxide red treated by 6% of perfluorooctyl triethoxysilane+2% of hydrogenated lecithin (embodiment 3) | 0.08 | Self-produced |
| | Iron oxide black treated by 6% of perfluorooctyl triethoxysilane+2% of hydrogenated lecithin (embodiment 4) | 0.02 | Self-produced |
| | Nylon 12 | 9.80 | Combined micro powder |
| B | PMX-0345 Fluid | 2.00 | Dow Corning |
| | PMX-200 Fluid, 5cst | 2.00 | Dow Corning |
| C | Euxyl PE 9010 | 0.60 | Schulke&Mayr |

### 2. Preparation process

steps: sequentially adding raw materials in phase A into a stirrer, and stirring and mixing at a high speed for 1 minute to uniformly mix different powders;
sequentially adding raw materials in phase B and C into an oil injection apparatus of the stirrer, and stirring and mixing at a high speed for 1 minute while injecting oil to fully and uniformly mix all the raw materials, and discharging.

Thus, the "loose powder A" is obtained; and if the loose powder is weighed in a pressed powder mold and pressed by a powder presser, a pressed powder can be obtained.

### Embodiment 8 (blusher preparation)

The powder prepared in embodiments 1-6 is added into a formula shown in Table 2 to prepare a blusher.

**Table 2 Blusher formula**

| Phase | Component name | Mass percentage % | Producer/supplier |
|---|---|---|---|
| A | Talcum powder treated by 6% of perfluorooctyl triethoxysilane+2% of hydrogenated lecithin (embodiment 5) | 45.10 | Self-produced |
| | Mica powder treated by 6% of perfluorooctyl triethoxysilane+2% of hydrogenated lecithin (embodiment 6) | 35.00 | Self-produced |
| | Magnesium stearate | 5.00 | Combined micro powder |
| D | Polysynlane LITE | 2.50 | NOF |
| | PMX-200 Fluid, 100cst | 2.50 | Dow Corning |
| | Indopol H-300 | 1.80 | INEOS |
| | Softisan 649 | 1.20 | Sasol |
| E | Euxyl PE 9010 | 0.60 | Schulke&Mayr |
| | Vitamin E-Acetate Care | 0.20 | BASF |
| B | Titanium dioxide treated by 6% of perfluorooctyl triethoxysilane+2% of hydrogenated lecithin (embodiment 1) | 2.00 | Self-produced |
| | Iron oxide yellow treated by 6% of perfluorooctyl triethoxysilane+2% of hydrogenated lecithin (embodiment 2) | 0.60 | Self-produced |
| | Iron oxide red treated by 6% of perfluorooctyl triethoxysilane+2% of hydrogenated lecithin (embodiment 3) | 2.00 | Self-produced |
| | Red 6 color lake | 0.50 | Sun Chemical |
| | Red 7 color lake | 1.00 | Sun Chemical |

### 2. Preparation process

steps: sequentially adding raw materials in phase A and raw materials in phase E into a stirrer, and stirring and mixing at a high speed for 1 minute;
adding raw materials in phase B into the stirrer, and stirring at a high speed for 3 minutes for uniformly mixing, thereby obtaining a mixture of the phase A, the phase E and the phase B;
pre-dissolving the phase D and uniformly stirring, then adding the mixture into an oil injection apparatus of the stirrer, and stirring and mixing at a high speed for 1 minute while injecting oil to fully and uniformly mix all the raw materials, and discharging; and
pressing the powder in an aluminum disk or a plastic shell, thereby obtaining the blusher.

### Embodiment 9

The powder prepared in embodiments 1, 2, 3 and 4 is added into a formula of the following liquid foundation (cream foundation) shown in Table 3.

**Table 3 Formula composition of liquid foundation (cream foundation)**

| Phase | Component name | Mass percentage % | Producer/supplier |
|---|---|---|---|
| A | Abil EM90 | 2.00 | Evonik |
| | KF-6017 | 1.00 | ShinEtsu |
| | 556 Cosmetic Grade Fluid | 7.50 | Dow Corning |
| | Silfoft 034 | 4.00 | Momentive |
| | 10# white oil | 3.00 | Hanglian |
| | 38V Gel | 3.00 | Self-prepared |
| | PMX-0345 Fluid | 3.90 | Dow Corning |
| | Cetiol SN | 1.45 | BASF |
| | Tegosoft TN | 4.35 | Evonik |
| | Liponate TDTM | 0.50 | Liponic |
| B | 593 Fluid | 1.00 | Dow Corning |
| C | Titanium dioxide treated by 6% of perfluorooctyl triethoxysilane+2% of hydrogenated lecithin (embodiment 1) | 9.18 | Self-produced |
| | Iron oxide yellow powder treated by 6% of perfluorooctyl triethoxysilane+2% of hydrogenated lecithin (embodiment 2) | 0.54 | |
| | Iron oxide red powder treated by 6% of perfluorooctyl triethoxysilane+2% of hydrogenated lecithin (embodiment 3) | 0.18 | |
| | Iron oxide black powder treated by 6% of perfluorooctyl triethoxysilane+2% of hydrogenated lecithin (embodiment 4) | 0.1 | |
| D | Deionized water | To 100 | Self-produced |
| | 1, 3-butanediol | 7.00 | Sansense |
| | Propylene glycol | 6.00 | Sansense |
| | Glycine betaine | 2.00 | Sansense |
| | Magnesium sulfate heptahydrate | 1.00 | Dicheng |
| E | Euxyl PE 9010 | 0.60 | Schulke&Mayr |
| | Essence | Appropriate | Givaudan |

### 2. Preparation process

steps: preparing 38V Gel: 20% Bentone 38 V(Hemmings)+ 75%PMX-0345 Fluid + 5% 95° alcohol, rapidly and uniformly stirring, and then homogenizing for 5 minutes;
stirring and heating the phase A to be about 70°, and homogenizing (at a rate of 10000 revolutions per minute) for 30 seconds after the components are completely dissolved;
adding the powder in the phase C, homogenizing (at the rate of 10000 revolutions per minute) for 1 minute, and uniformly dispersing the powder;
adding the phase B into the phases A and C, rapidly stirring the phases A, B and C (at a rate of 400 revolutions per minute), and simultaneously heating the phase D to be about 75-80° until the components are completely dissolved;
maintaining the temperature, increasing the stirring speed (400-500 revolutions per minute) of the phases A, B and C, slowly adding the phase D into the phases A, B and C to fully complete emulsification, and homogenizing (at the rate of 10000 revolutions per minute) for 1 minute; and
stirring (at a rate of 380-400 revolutions per minute), cooling to a temperature below 45°, adding the phase E, and uniformly stirring and discharging.

### TEST CASE 1

Contrast case 1: steps: dissolving 60g of perfluorooctyl triethoxysilane (Dynasylan F8261, Evonik Degussa) in 100g of ethanol to prepare a treatment solution; spraying the treatment solution into 1kg of titanium dioxide while high-speed stirring, fully mixing the treatment solution and powder and then discharging, and drying at 110° for 3h; and performing air jet milling after discharging, thereby obtaining the titanium dioxide subjected to fluorination treatment.

Contrast case 2: the titanium dioxide in the contrast case 1 is replaced with iron oxide yellow.

Contrast case 3: the titanium dioxide in the contrast case 1 is replaced with iron oxide red.

Contrast case 4: the titanium dioxide in the contrast case 1 is replaced with iron oxide black.

Contrast case 5: the titanium dioxide in the contrast case 1 is replaced with talcum powder.

Contrast case 6: the titanium dioxide in the contrast case 1 is replaced with sericite.

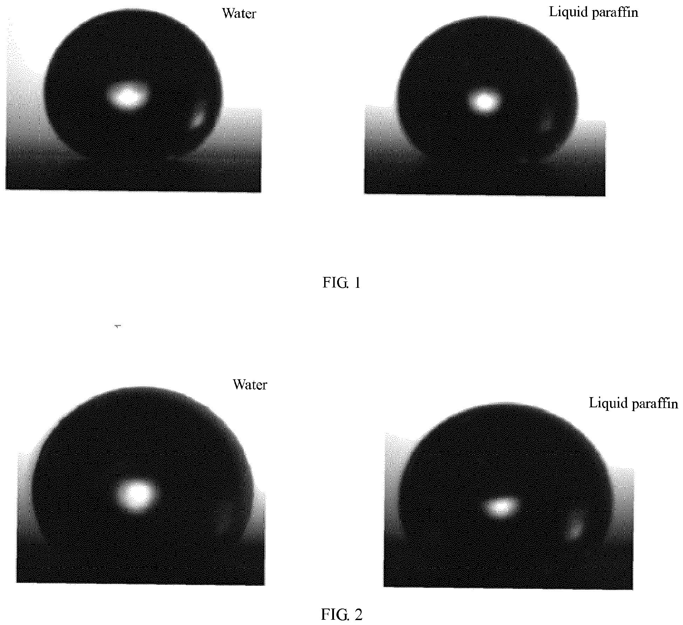

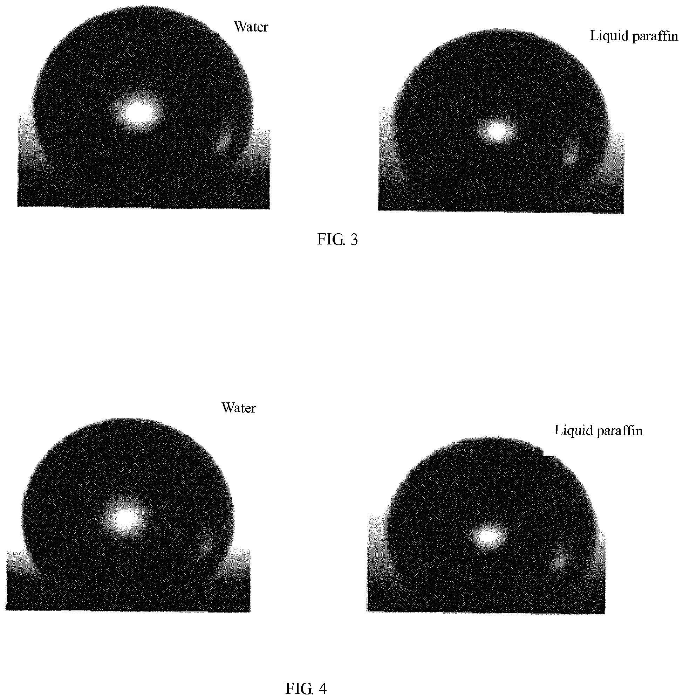

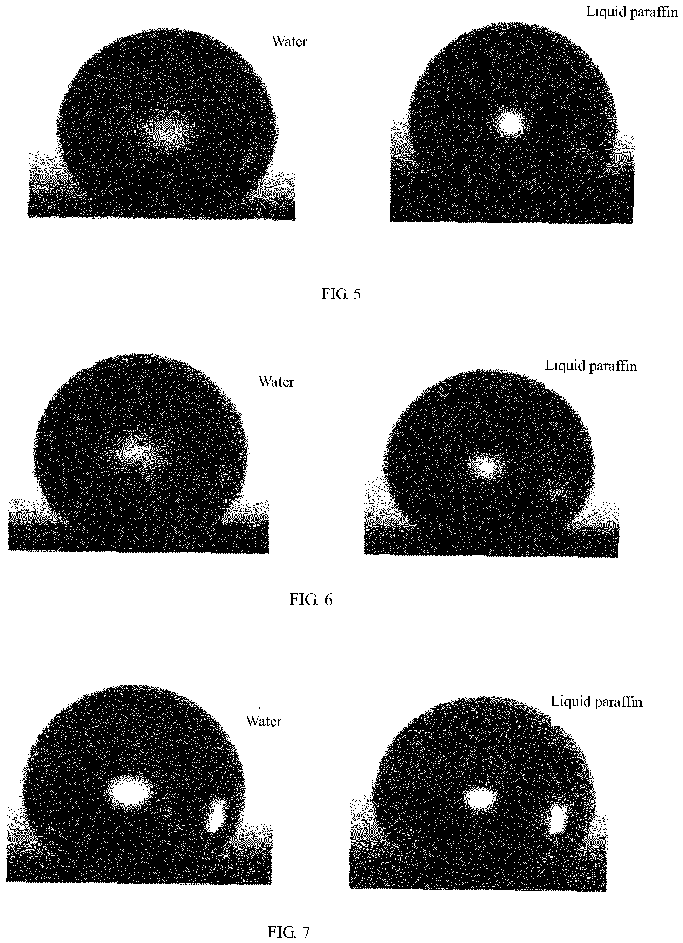

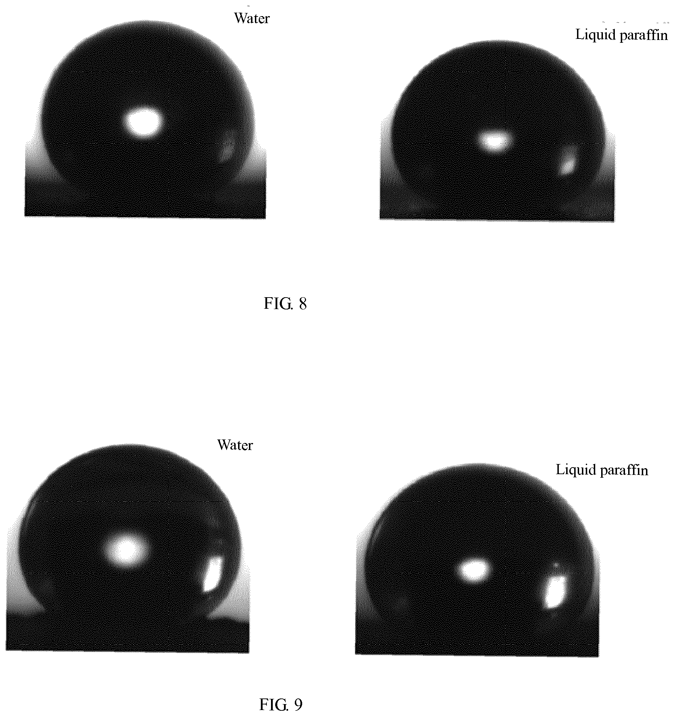

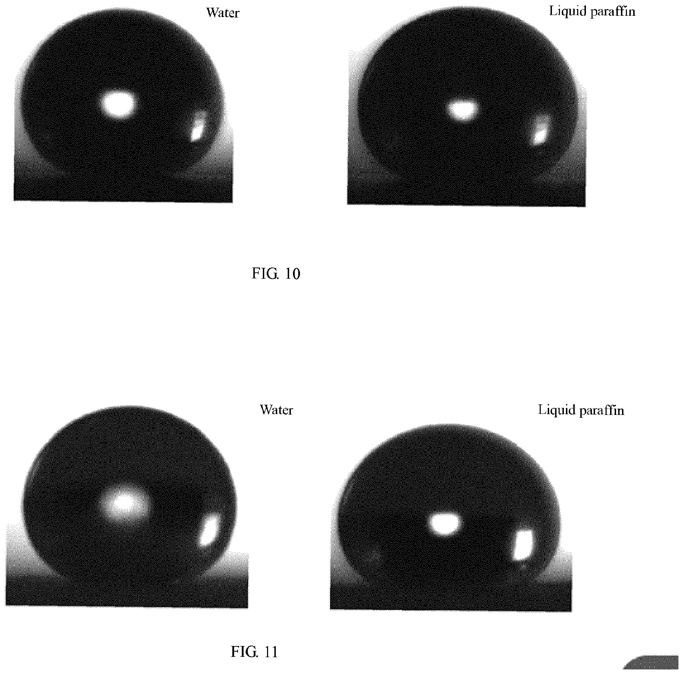

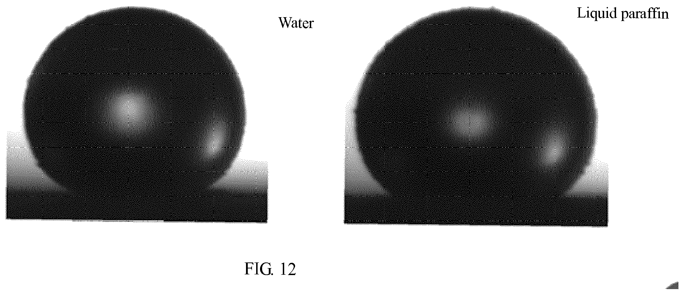

Powder prepared in embodiments 1, 2, 3, 4, 5 and 6 and the contrast cases 1, 2, 3, 4, 5 and 6 is subjected to contact angle test, and test fluid is respectively deionized water and liquid paraffin. Test results are shown in Fig. 1-Fig. 12 (water on the left side, and liquid paraffin on the right side) and Table 4.

**Table 4 Contact angle test performed on the powder prepared in embodiments 1, 2, 3 and 4 and contrast cases 1, 2, 3 and 4**

| Powder name (embodiment) | Contact angle of water (°) | Contact angle of liquid paraffin (°) |
|---|---|---|
| Titanium dioxide treated by 6% of perfluorooctyl triethoxysilane+2% of hydrogenated lecithin (embodiment 1) | 151 | 147 |
| Iron oxide yellow powder treated by 6% of perfluorooctyl triethoxysilane+2% of hydrogenated lecithin (embodiment 2) | 139 | 129 |
| Iron oxide red powder treated by 6% of perfluorooctyl triethoxysilane+2% of hydrogenated lecithin (embodiment 3) | 145 | 129 |
| Iron oxide black powder treated by 6% of perfluorooctyl triethoxysilane+2% of hydrogenated lecithin (embodiment 4) | 143 | 135 |
| Talcum powder treated by 6% of perfluorooctyl triethoxysilane+2% of hydrogenated lecithin (embodiment 5) | 150 | 136 |
| Mica powder treated by 6% of perfluorooctyl triethoxysilane+2% of hydrogenated lecithin (embodiment 6) | 145 | 140 |
| Titanium dioxide treated by 6% of perfluorooctyl triethoxysilane (contrast case 1) | 156 | 150 |
| Iron oxide yellow powder treated by 6% of perfluorooctyl triethoxysilane (contrast case 2) | 140 | 130 |
| Iron oxide red powder treated by 6% of perfluorooctyl triethoxysilane (contrast case 3) | 146 | 132 |
| Iron oxide black powder treated by 6% of perfluorooctyl triethoxysilane (contrast case 4) | 147 | 140 |
| Talcum powder treated by 6% of perfluorooctyl triethoxysilane (contrast case 6) | 155 | 140 |
| Mica powder treated by 6% of perfluorooctyl triethoxysilane (contrast case 7) | 150 | 148 |

Conclusion: the hydrophobic and oleophilic properties of the powder subjected to the compounding treatment of the hydrogenated lecithin are not decreased, so the powder has excellent make-up retaining property.

### TEST CASE 2

The powder treated by 6% of perfluorooctyl triethoxysilane prepared in the contrast cases 2, 3 and 4 replaces the powder treated by 6% of perfluorooctyl triethoxysilane+2% of hydrogenated lecithin prepared in embodiments 2, 3 and 4 and is added into the formula of the pressed powder (loose powder) shown in Table 2, and sample making is performed according to the same process, thereby obtaining "loose powder B".

A foundation evaluation method: searching several volunteers, respectively applying the "foundation A" and the "foundation B" to left and right cheeks, evaluating elasticity, smoothness and application property during application, and evaluating skin-fitting properties and make-up retaining effects of the two kinds of foundations after application. Contrast results are shown in Table 5.

**Table 5 Skin feeling contrast results of the loose powder A and B**

| Number of loose powder | | Evaluation indexes | | | | |
|---|---|---|---|---|---|---|
| | Make-up retaining effect after 8 hours | Skin-fitting property | Elasticity | Smoothness | Applicatio n property | Easiness degree of make-up removal |
| Loose powder A(embodiment 7) | 10 | 10 | 10 | 10 | 10 | 9 |
| Loose powder B(embodiment 7) | 10 | 5 | 6 | 8 | 8 | 5 |

| | | | | | | |
|---|---|---|---|---|---|---|
| Note: The full mark is 10. | | | | | | |

Conclusion: the powder treated by 6% of perfluorooctyl triethoxysilane+2% of hydrogenated lecithin in the present invention is better than powder treated without the hydrogenated lecithin in the aspects of skin-fitting property, elasticity, smoothness, application property and easiness degree of make-up removal.

### TEST CASE 3 Type screening test of treatment agent used with perfluorooctyl triethoxysilane

Amino acid, lecithin and hydrogenated lecithin belong to substances with biocompatibility with skin. Therefore, an appropriate treatment agent is screened from the three types of skin-fitting treatment agents.

High-purity lecithin has strong oxidation property and is extremely easy to be oxidized, so non-hydrogenated lecithin is generally required to be placed in a nitrogen-filled sealed container and is extremely inconvenient in use.

The amino acid is generally treated by a wet process. Treatment cost is high, and lots of waste water may be discharged.

The method includes the steps: dissolving 50g of lecithin in 100g of ethanol to prepare a treatment solution; spraying the treatment solution into 1kg of titanium dioxide while high-speed stirring, fully mixing the treatment solution and powder and then discharging, and drying at 110° for 3h; and performing air jet milling after discharging, thereby obtaining titanium dioxide treated by 5% of lecithin.

The above lecithin is replaced with the hydrogenated lecithin, and the same process is performed, thereby obtaining titanium dioxide treated by 5% of hydrogenated lecithin.

The method includes the steps: pre-dispersing 1kg of titanium dioxide into a 16%-20% of aqueous solution, regulating a pH value of the solution to be about 11 by using sodium hydroxide, and adding 50g of lauryl lysine powder; raising a temperature of the solution to 80°, stirring and mixing for 40 minutes, regulating the pH value to be 6 by using hydrochloric acid, maintaining the temperature, stirring to react for 4 minutes, washing, drying and grinding, thereby obtaining the titanium dioxide treated by 5% of amino acid.

By comparing the skin-fitting properties of the powders obtained by the three treatment methods, it is discovered that, the skin-fitting properties of the powders treated by the lecithin and the hydrogenated lecithin are better than that of the powder treated by the amino acid. However, since the lecithin is extremely easy to be oxidized and is inconvenient in use, the hydrogenated lecithin is selected as the skin-fitting treatment agent.

### TEST CASE 4 Dosage screening test of treatment agent used with perfluorooctyl triethoxysilane

A treatment ratio of the perfluorooctyl triethoxysilane to the hydrogenated lecithin in the patent is from 1:1 to 15:1, and preferably 3:1. A screening test is as follows:
fixing a dosage of the perfluorooctyl triethoxysilane according to an experimental process in embodiment 1, and changing a dosage of the hydrogenated lecithin to change a proportional relation between the two treatment agents; and respectively adding 70g, 60g, 20g, 4g and 3g of the hydrogenated lecithin, and correspondingly changing the dosage of the ethanol to be 150g, 130g, 100g, 80g and 80g, thereby obtaining the titanium dioxide with compounding treatment ratios of 6:7, 1:1, 3:1, 5:1 and 20:1 of the perfluorooctyl triethoxysilane to the hydrogenated lecithin.

When it is discovered that the dosage of the hydrogenated lecithin is greater than that of the perfluorooctyl triethoxysilane later, i.e., when the treatment ratio is 6:7, the treated powder is poor in oleophobic property (a contact angle of liquid paraffin is less than 90 degrees). When the dosage of the hydrogenated lecithin is too small, i.e., when the compounding treatment ratio of the perfluorooctyl triethoxysilane to the hydrogenated lecithin is above 15:1, the powder loses elastic-smooth feeling and excellent skin-fitting property. Therefore, the treatment ratio of the perfluorooctyl triethoxysilane to the hydrogenated lecithin is selected within a range of 1:1 to 15:1, and in the range, both hydrophobic and oleophobic properties may be ensured, and the elastic-smooth feeling and the skin-fitting property may be also ensured. Further, when the treatment ratio of the perfluorooctyl triethoxysilane to the hydrogenated lecithin is 3:1, the hydrophobic and oleophobic properties and the elastic-smooth feeling are the best.

The above description of specific exemplary embodiments of the present invention is for the purpose of explaining and illustrating. The description is not intended to limit the present invention to a disclosed precise form, and apparently, many modifications and changes may be made according to the above teaching. A purpose for selecting and describing the exemplary embodiments is to explain a specific principle of the present invention and actual applications thereof. Therefore, those skilled in the art may realize and utilize various exemplary embodiments and various selections and modifications in the present invention. A scope of the present invention is limited by claims and equivalent forms thereof.

## Claims

1. A hydrophobic and oleophobic cosmetic pigment powder, comprising a pigment powder and a skin-fitting treatment agent, wherein the skin-fitting treatment agent performs surface compounding treatment on the pigment powder and comprises fluorine-containing silane and hydrogenated lecithin.

2. The hydrophobic and oleophobic cosmetic pigment powder according to claim 1, wherein the fluorine-containing silane and the hydrogenated lecithin in the skin-fitting treatment agent are used together according to a weight ratio of 1:1-15:1.

3. The hydrophobic and oleophobic cosmetic pigment powder according to claim 1, wherein the hydrophobic and oleophobic cosmetic pigment powder comprises 0.1-10.0% of the fluorine-containing silane, 0.1-10.0% of the hydrogenated lecithin, and the balance of pigment powder in percentage by weight.

4. The hydrophobic and oleophobic cosmetic pigment powder according to claim 1, wherein the pigment powder includes but not limited to one or a mixture of more of titanium dioxide, iron oxide yellow, iron oxide red, iron oxide black, talc, mica, ultramarines and chromium oxide green.

5. The hydrophobic and oleophobic cosmetic pigment powder according to claim 1, wherein a structure of the fluorine-containing silane is R_{f}-(CH₂)₂-Si-(OCₘH₂ₘ₊₁)₃, wherein R_{f} is CₙF₂ₙ₊₁, n is more than or equal to 1 and less than or equal to 6, n is an integer, and m is 1 or 2.

6. The hydrophobic and oleophobic cosmetic pigment powder according to claim 1, wherein the fluorine-containing silane is perfluorooctyl triethoxysilane.

7. The hydrophobic and oleophobic cosmetic pigment powder according to claim 1, wherein the hydrophobic and oleophobic cosmetic pigment powder is composed of the following substances in percentage by weight:
99.64% of talcum powder treated by 6% of perfluorooctyl triethoxysilane+2% of hydrogenated lecithin;
0.24% of iron oxide yellow treated by 6% of perfluorooctyl triethoxysilane+2% of hydrogenated lecithin;
0.10% of iron oxide red treated by 6% of perfluorooctyl triethoxysilane+2% of hydrogenated lecithin; and
0.02% of iron oxide black treated by 6% of perfluorooctyl triethoxysilane+2% of hydrogenated lecithin;
or
53.25% of talcum powder treated by 6% of perfluorooctyl triethoxysilane+2% of hydrogenated lecithin;
41.32% of mica powder treated by 6% of perfluorooctyl triethoxysilane+2% of hydrogenated lecithin;
2.36% of titanium dioxide treated by 6% of perfluorooctyl triethoxysilane+2% of hydrogenated lecithin;
0.71% of iron oxide yellow treated by 6% of perfluorooctyl triethoxysilane+2% of hydrogenated lecithin; and
2.36% of iron oxide red treated by 6% of perfluorooctyl triethoxysilane+2% of hydrogenated lecithin;
or
91.8% of titanium dioxide treated by 6% of perfluorooctyl triethoxysilane+2% of hydrogenated lecithin;
5.4% of iron oxide yellow treated by 6% of perfluorooctyl triethoxysilane+2% of hydrogenated lecithin;
1.8% of iron oxide red treated by 6% of perfluorooctyl triethoxysilane+2% of hydrogenated lecithin; and
1.0% of iron oxide black treated by 6% of perfluorooctyl triethoxysilane+2% of hydrogenated lecithin.

8. An application of the hydrophobic and oleophobic cosmetic pigment powder of any one of claims 1-7 in cosmetics.

9. A preparation method of the hydrophobic and oleophobic cosmetic pigment powder according to claim 1, comprising the following steps:
step (1), preparing a skin-fitting treatment solution: dissolving the fluorine-containing silane and the hydrogenated lecithin in ethanol or polyhydric alcohols to prepare the treatment solution;
step (2), spraying the treatment solution onto a surface of the pigment powder while stirring at high speed, uniformly mixing and discharging, heating and drying; and
step (3), performing air jet milling to obtain the powder.

10. The preparation method of the hydrophobic and oleophobic cosmetic pigment powder according to claim 9, wherein the preparation method comprises the following steps:
step (1), preparing a skin-fitting treatment solution: dissolving the fluorine-containing silane and the hydrogenated lecithin in ethanol or polyhydric alcohols with an amount of 1-1.5 times while stirring at a high speed of 2000-20000 revolutions per minute to prepare a treatment solution;
step (2), spraying the treatment solution onto a surface of the pigment powder while stirring at the high speed of 2000-20000 revolutions per minute, uniformly mixing and discharging, heating at 100-150° and drying for 1-5 hours; and
step (3), performing air jet milling: treating the powder by adopting high-pressure air of 7-8 Mpa, thereby obtaining the powder with a mean particle size of 0.1-5µm.
